(19) Europäisches Patentamt
European Patent Office
Office européen des brevets

(11) **EP 4 190 306 A1**

(12) **EUROPEAN PATENT APPLICATION**
published in accordance with Art. 153(4) EPC

(43) Date of publication:
**07.06.2023 Bulletin 2023/23**

(21) Application number: **21849346.8**

(22) Date of filing: **27.07.2021**

(51) International Patent Classification (IPC):
**A61K 8/365** (2006.01)      **A61K 8/67** (2006.01)
**A61K 8/73** (2006.01)       **A61K 8/81** (2006.01)
**A61Q 5/08** (2006.01)

(52) Cooperative Patent Classification (CPC):
**A61K 8/365; A61K 8/67; A61K 8/73; A61K 8/81;
A61Q 5/08**

(86) International application number:
**PCT/JP2021/027670**

(87) International publication number:
**WO 2022/025032 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.07.2020 PCT/JP2020/028665**

(71) Applicant: **Kao Corporation
Chuo-ku
Tokyo 103-8210 (JP)**

(72) Inventors:
• **CHEN, Ying tzu
Hsinchu Hsien 30352 (TW)**
• **MORISHIMA, Atsumi
Tokyo 131-8501 (JP)**
• **WANG, Shu fen
Hsinchu Hsien 30352 (TW)**
• **OGAWA, Toshio
Tokyo 131-8501 (JP)**

(74) Representative: **Hoffmann Eitle
Patent- und Rechtsanwälte PartmbB
Arabellastraße 30
81925 München (DE)**

(54) **HAIR TREATMENT METHOD**

(57)     The present invention provides a hair treatment method comprising the following step 1 and step 2, the step 2 being carried out after the step 1:
(step 1) applying to the scalp a cosmetic composition comprising a pH buffer, an antioxidant and a pH-responsive thickener and having a viscosity of from 1 to 30,000 mPa·s at pH 4.1; and
(step 2) applying to the hair at least one hair treatment agent composition selected from the group consisting of a hair color composition, a hair bleach composition, a perm composition and a relaxer composition. The hair treatment method improves the scalp protection ability and enables maintenance of a desired hair treatment effect. The cosmetic composition used has a good balance between an application property and a dripping property, so that it is possible to improve convenience of use for users.

EP 4 190 306 A1

**Description**

Field of the Invention

The present invention relates to a hair treatment method, and particularly to a hair treatment method for protecting a scalp against the damage from a hair color composition, a hair bleach composition, a perm composition or a relaxer composition.

Background of the Invention

Hair treatment such as hair coloring, hair bleaching, perming or relaxation (e.g. hair straightening) can change the appearance of a person, so that the person can feel confident. However, a hair treatment agent such as a hair colorant, a hair bleach, a perm chemical, and a relaxer may cause problems such as damage to the hair and irritation to the scalp. Thus, in this technical field, hair treatment methods for protecting the scalp against the damage from a hair color composition, a hair bleach composition, a perm composition or a relaxer composition have been developed.

For example, Patent Literature 1 discloses a scalp isolation composition which is used before hair coloring and perming, the scalp isolation composition containing a nonionic surfactant, a water-soluble polymer, an antioxidant, and a pH buffer; and a method for use thereof.

Citation List

Patent Literature

(Patent Literature 1) CN 107837212 A

Summary of the Invention

The present invention provides a hair treatment method comprising the following step 1 and step 2, the step 2 being carried out after the step 1:

(step 1) applying to a scalp a cosmetic composition comprising the following components (A) to (C) and having a viscosity of from 1 to 30,000 mPa·s at pH 4.1 (the pH is a "pH at 25°C" unless otherwise specified herein):

(A) a pH buffer;
(B) an antioxidant; and
(C) a pH-responsive thickener; and

(step 2) applying to the hair at least one hair treatment composition selected from the group consisting of a hair color composition, a hair bleach composition, a perm composition and a relaxer composition.

The present invention also provides a cosmetic composition for protecting a scalp, comprising the following components (A) to (C) and having a viscosity of from 1 to 30,000 mPa·s at pH 4.1:

(A) a pH buffer;
(B) an antioxidant; and
(C) a pH-responsive thickener.

The present invention also provides a kit containing at least one or more hair treatment compositions selected from the group consisting of a hair color composition, a hair bleach composition, a perm composition and a relaxer composition, and the cosmetic composition of the present invention described above.

Detailed Description of the Invention

The composition and the method for use thereof which are disclosed in Patent Literature 1 reduce or alleviate damage from a hair treatment agent such as a hair colorant, but these still have room for improvement in scalp protection. Further, studies have revealed that the composition of Patent Literature 1 reduces the dyeability of a hair colorant, which is problematic.

The present inventors have energetically conducted studies, and resultantly found that when a cosmetic composition containing a pH buffer, an antioxidant and a pH-responsive thickener and having a viscosity of from 1 to 30,000 mPa·s at pH 4.1 is applied before application of a hair treatment agent composition, the scalp protection ability can be significantly improved, and a desired hair treatment effect such as hair coloring, hair bleaching, perming or relaxation can be maintained, so that it is possible to solve the problem described above.

As is apparent from a certain embodiment of the present invention, the hair treatment method of the present invention includes applying a cosmetic composition before carrying out hair treatment, and therefore can significantly improve the scalp protection ability, so that damage to the scalp from the hair treatment agent composition can be reduced or alleviated. The cosmetic composition used ensures that even after application thereof to the scalp, a desired hair treatment effect does not deteriorate, and can be maintained at a good level. For example, when a hair color composition is applied, an excellent dyeing effect can be maintained. The present invention enables maintenance of a good balance between an application property and a dripping property of the cosmetic composition, so that convenience of use for users can be improved.

Hereinafter, embodiments of the present invention will be described in detail.

The present invention provides a hair treatment (e.g. hair coloring, hair bleaching, perming or relaxation) method including uniformly applying a cosmetic composition of the present invention to the scalp, applying a hair treatment agent composition (e.g. a composition such as a hair colorant, a hair bleach, a perm chemical or a relaxer) to the hair, and then optionally rinsing and drying the hair.

Specifically, the hair treatment method of the present invention includes the following step 1 and step 2, the step 2 being carried out after the step 1:

(step 1) applying to the scalp a cosmetic composition containing the following components (A), (B) and (C) and having a viscosity of from 1 to 30,000 mPa·s at pH 4.1:

(A) a pH buffer;
(B) an antioxidant; and
(C) a pH-responsive thickener; and

(step 2) applying to the hair at least one hair treatment agent composition selected from the group consisting of a hair color composition, a hair bleach composition, a perm composition and a relaxer composition.

In the present invention, from the viewpoint of obtaining an effect that is an excellent scalp protection ability, a cosmetic composition for use in step 1 is applied before application of a hair treatment agent composition, whereby the scalp can be protected against damage from hair treatment. Since the cosmetic composition does not affect hair treatment, an excellent hair treatment effect can be maintained.

Examples of the hair treatment include hair treatments including chemical treatment with an alkaline treatment agent, and specific examples thereof include hair treatments such as hair coloring, hair bleaching, perming and relaxation.

Here, the relaxation refers to hair treatment in which by treating curly hair under a strong alkaline condition using a hair treatment agent containing an alkali agent such as sodium hydroxide or guanidine hydroxide, the hair is relaxed and straightened.

The perming refers to hair treatment in which cystine bonds in the hair are cleaved with a first liquid containing a reducing substance such as a thioglycolic acid salt or cysteine, or an alkali agent, to thereby swell and soften the hair, and the hair is wound around a rod or the like (wave perm), or linearly extended with a comb, an iron or the like (straight perm) to set the hair in a desired shape, and cystine bonds are then re-formed with a second liquid containing an oxidizing agent such as a bromic acid salt or hydrogen peroxide to fix the hair in a desired shape. There exist not only alkaline perms using an alkaline perming first liquid as described above but also acidic or neutral perms using glyceryl thioglycolate, butyrolactonethiol or the like as a reducing substance.

The hair coloring refers to hair treatment in which a first part containing an alkali agent and an oxidation dye intermediate is mixed with a second part containing an oxidizing agent such as hydrogen peroxide, and the resulting mixture is applied to the hair to dye the hair.

The hair bleaching refers to hair treatment in which a first part containing an alkali agent is mixed with a second part containing an oxidizing agent such as hydrogen peroxide, and the resulting mixture is applied to the hair to bleach the hair.

For hair coloring and hair bleaching, a granulated product of a persulfuric acid salt or the like may be further used in combination with the above for improving the bleaching ability.

In step 1, the cosmetic composition only needs to be applied to the scalp. The cosmetic composition may be applied to a dry scalp, or a wet scalp. Since the cosmetic composition of the present invention does not significantly affect the hair treatment in step 2, and does not irritate the scalp, a part of the cosmetic composition may be applied to the vicinity of the root of the hair. The area to which the cosmetic composition is applied is not limited as long as the hair treatment agent composition can contact the scalp in step 2, and it may be the entire scalp or a part of the scalp. From the viewpoint of obtaining a sufficient scalp protection effect, the amount of the cosmetic composition applied to the scalp is preferably in the range of from 8 to 40 $\mu$L/cm$^2$, more preferably in the range of from 13 to 27 $\mu$L/cm$^2$ or in the range of from 20 to 27 $\mu$L/cm$^2$.

The above hair treatment may include pretreatment with a hair cosmetic as described in JP-A-2019-123710, or the like between step 1 and step 2, as appropriate, but does not include a step of washing off the cosmetic composition for use in step 1. The timing at which the hair treatment in step 2 is carried out is not particularly limited, and the hair treatment may be carried out immediately after or several minutes to several hours after the cosmetic composition for use in step 1 is applied to the scalp. From the viewpoint of maintaining a desired hair treatment effect and obtaining a sufficient scalp protection effect, it is preferable that step 2 be carried out immediately after the cosmetic composition for use in step 1 is applied to the scalp.

The mass of the hair treatment agent composition applied to the hair in step 2 in terms of a bath ratio to the mass of the hair [(mass of the hair treatment agent composition)/(mass of the hair)] is preferably 0.05 or more, more preferably 0.10 or more, further more preferably 0.25 or more, further more preferably 0.5 or more, and preferably 100 or less, more preferably 90 or less, further more preferably 40 or less, further more preferably 5 or less, further more preferably 3 or less, further more preferably 2 or less.

In step 2, the treatment period of time is preferably 1 minute or more, more preferably 3 minutes or more, further more preferably 5 minutes or more, and preferably 1 hour or less, more preferably 30 minutes or less, further more preferably 20 minutes or less, from the viewpoint of promoting permeation of the hair treatment agent composition to enhance the treatment effect. Here, heating may be performed, from the viewpoint of promoting permeation of the first part. When heating is performed, it is preferable to perform the heating at from 40 to 90°C.

After step 2, the hair may be optionally rinsed with water, and washed with shampoo and rinsed, as appropriate. Further, the hair may be subjected to treatment with a rinse, a conditioner, or the like, and rinsed. It is preferable that thereafter, drying with a towel be performed as appropriate to remove excess moisture, followed by drying.

Hereinafter, the cosmetic composition for use in step 1 will be described.

(Component (A))

The component (A) is a pH buffer, and is not particularly limited. The component (A) is preferably a buffer solution of a conjugate acid-base pair. Specific examples thereof include monocarboxylic acids such as acetic acid and propionic acid, and salts thereof; dicarboxylic acids such as malonic acid, succinic acid, glutaric acid, adipic acid, maleic acid, fumaric acid and phthalic acid, and salts thereof; polycarboxylic acids such as polyglutamic acid and salts thereof; hydroxycarboxylic acids such as glycolic acid, lactic acid, hydroxyacrylic acid, glyceric acid, malic acid, tartaric acid and citric acid, and salts thereof; acidic amino acids such as glutamic acid and aspartic acid and salts thereof; inorganic acids such as hydrochloric acid, phosphoric acid and sulfuric acid, and salts thereof; and barbituric acid, and salts thereof. The salt of the acid described above is not particularly limited, and examples thereof include alkali metal salts such as sodium salts and potassium salts. These pH buffers may be used singly, or in combination of two or more thereof.

For hair treatment such as hair coloring, an alkaline hair treatment agent is typically used, and therefore the pH of the scalp is likely to increase during the hair treatment, resulting in an irritating sensation. Thus, from the viewpoint of suppressing an increase in pH of the scalp, it is preferable to use a pH buffer having a buffering ability at least over a pH range of from 4.5 to 5.5.

It is preferable that as the pH buffer having a buffering ability at least over a pH range of from 4.5 to 5.5, an acid with a pKa (25°C) of from 4.0 to 6.5 and a salt thereof be used to perform adjustment, and specific examples of the acid include barbituric acid (pKa1 = 4.04), succinic acid (pKa1 = 4.21 and pKa2 = 5.64), citric acid (pKa2 = 4.76 and pKa3 = 6.40), acetic acid (pKa2 = 4.76), malic acid (pKa2 = 5.13), phthalic acid (pKa2 = 5.41), and carbonic acid (pKa2 = 6.35).

In particular, from the viewpoints of improving the scalp protection ability, maintaining a good balance between an application property and a dripping property, and maintaining an desired hair treatment effect, the component (A) is preferably succinic acid and a salt thereof, a malic acid and a salt thereof, a citric acid and a salt thereof, or any combination of the acids and a salt thereof, more preferably a combination of citric acid and a salt thereof, further more preferably a combination of citric acid and sodium citrate.

From the viewpoints of improving the scalp protection ability, and maintaining a desired hair treatment effect, the content of the component (A) (total content of a conjugate acid-base pair when the component (A) is the conjugate acid-base pair) is, for example, more than 0.15 mass%, preferably 0.2 mass% or more, more preferably 0.5 mass% or more, further more preferably 1.0 mass% or more, even more preferably 1.5 mass% or more, based on the total amount of the cosmetic composition. From the same viewpoints, the content of the component (A) is, for example, 10.0 mass% or less, preferably 9.0 mass% or less, more preferably 8.0 mass% or less, further more preferably 6.0 mass% or less, even more preferably 4.0 mass% or less.

More specifically, from the above-described viewpoints, the content of the component (A) is, for example, more than 0.15 mass% and 10.0 mass% or less, preferably 0.2 mass% or more and 9.0 mass% or less, more preferably 0.5 mass% or more and 8.0 mass% or less, further more preferably 1.0 mass% or more and 6.0 mass% or less, even more preferably 1.0 mass% or more and 4.0 mass% or less, even more preferably 1.5 mass% or more and 4.0 mass% or less.

(Component (B))

The component (B) is an antioxidant, and is not particularly limited, and examples thereof include dibutylhydroxytoluene, butylhydroxyanisole, vitamin E such as delta-tocopherol or derivatives thereof (specific examples thereof include tocopherol acetate), thiotaurine, thioglycolic acid, propanoic acid, tert-butylhydroquinone, nicotinic acid or derivatives thereof, catechin, *Oenothera biennis* extract, beta-carotene, flavonoid compounds, sulfurous acid or salts thereof, ascorbic acid or salts or derivatives thereof (specific examples thereof include ascorbic acid glucoside, magnesium ascorbyl phosphate, erythorbic acid, and sodium erythorbate), L-cysteine or salts or derivatives thereof, N-acetyl-L-cysteine or salts or derivatives thereof, and phenol-based antioxidants such as polyphenol compounds. These antioxidants may be used singly, or in combination of two or more thereof.

The salt of the acid described above is not particularly limited, and examples thereof include alkali metal salts such as sodium salts and potassium salts.

In particular, from the viewpoints of improving the scalp protection ability, maintaining a good balance between an application property and a dripping property, and maintaining a desired hair treatment effect, the component (B) is preferably ascorbic acid or a salt or a derivative thereof, dibutylhydroxytoluene, or catechin, more preferably ascorbic acid or a salt or a derivative thereof (e.g. ascorbic acid glucoside, magnesium ascorbyl phosphate, erythorbic acid, or sodium erythorbate), further more preferably ascorbic acid or ascorbic acid glucoside.

From the viewpoints of improving the scalp protection ability, maintaining a good balance between an application property and a dripping property, and maintaining a desired hair treatment effect, the total content of the component (B) is, for example, 0.01 mass% or more, preferably 0.1 mass% or more, more preferably 0.2 mass% or more, based on the total amount of cosmetic composition. From the same viewpoints, the total content of the component (B) is, for example, 5.0 mass% or less, preferably 3.0 mass% or less, more preferably 1.5 mass% or less, further more preferably 1.0 mass% or less, even more preferably 0.7 mass% or less.

More specifically, from the above-described viewpoints, the total content of the component (B) is, for example, 0.01 mass% or more and 5.0 mass% or less, preferably 0.1 mass% or more and 3.0 mass% or less, more preferably 0.2 mass% or more and 1.5 mass% or less, further more preferably 0.2 mass% or more and 1.0 mass% or less, even more preferably 0.2 mass% or more and 0.7 mass% or less.

From the viewpoints of improving the scalp protection ability, and maintaining a desired hair treatment effect, the total content of the component (A) and the component (B) is, for example, preferably 0.2 mass% or more, more preferably 1.0 mass% or more, further more preferably 2.3 mass% or more, even more preferably 2.5 mass% or more. From the same viewpoints, the total content of the component (A) and the component (B) is, for example, 10.0 mass% or less, preferably 8.0 mass% or less, more preferably 6.0 mass% or less, further more preferably 5.5 mass% or less, even more preferably 5.0 mass% or less, even more preferably 4.5 mass% or less, even more preferably 4.0 mass% or less.

More specifically, from the above-described viewpoints, the total content of the component (A) and the component (B) is, for example, 0.2 mass% or more and 10.0 mass% or less, preferably 1.0 mass% or more and 8.0 mass% or less, more preferably 2.3 mass% or more and 6.0 mass% or less, further more preferably 2.3 mass% or more and 5.5 mass% or less, even more preferably 2.5 mass% or more and 5.0 mass% or less, even more preferably 2.5 mass% or more and 4.5 mass% or less, even more preferably 2.5 mass% or more and 4.0 mass% or less.

From the viewpoints of improving the scalp protection ability, and maintaining a desired hair treatment effect, a mass ratio of the component (A) to the component (B) [(component (A))/(component (B))] is, for example, 1.0 or more, preferably 1.1 or more, more preferably 1,5 or more, further more preferably 2.0 or more, even more preferably 3.0 or more. From the same viewpoints, the ratio is, for example, 8.0 or less, preferably 7.5 or less, more preferably 6.5 or less, further more preferably 6.0 or less, even more preferably 5.0 or less.

More specifically, from the above-described viewpoints, the ratio is, for example, 1.0 or more and 8.0 or less, preferably 1.1 or more and 7.5 or less, more preferably 1.5 or more and 6.5 or less, further more preferably 2.0 or more and 6.0 or less, even more preferably 3.0 or more and 5.0 or less.

(Component (C))

The component (C) is a pH-responsive thickener, and can increase the viscosity of the cosmetic composition in response to an increase in pH of the cosmetic composition. The component (C) is not particularly limited, and is preferably an ionic water-soluble polymer, more preferably an alkali thickening ionic water-soluble polymer, from the viewpoint of improving the scalp protection ability. When the above-described ionic water-soluble polymer is dispersed or dissolved in water, the viscosity does not significantly increase because dissociable groups contained are not dissociated or dissociated only in a small amount, but when a base such as sodium hydroxide, potassium hydroxide or triethanolamine is added, an effect is obtained such that the pH increases, dissociable groups are dissociated and ionized, and the repulsive force of charge generated causes swelling to increase the viscosity. It is preferable that the viscosity of the pH-responsive thickener increase in response to an increase in pH at least over a part or the whole of the pH range of from 3 to 7.

The above-described pH-responsive thickener is more preferably an ionic water-soluble polymer having at least one selected from the group consisting of an unneutralized carboxyl group and an unneutralized sulfonate group, further more preferably an ionic water-soluble polymer having at least an unneutralized carboxyl group. Examples thereof include linear or branched polymers such as acrylic acid, methacrylic acid, maleic acid, fumaric acid, itaconic acid, styrenesulfonic acid, vinylbenzoic acid, 2-acrylamido-2-methyl-1-propanesulfonic acid and dimethylolpropionic acid, or crosslinked polymers and copolymers thereof. Specific examples include acryl-based polymers or copolymers such as polyacrylic acid, carboxyvinyl polymers, sodium polyacrylate and acrylic acid/alkyl methacrylate copolymers; and methacrylic acid-based polymers or copolymers such as polymethacrylic acid and methacrylic acid/alkyl methacrylate copolymers. These pH-responsive thickeners may be used singly, or in combination of two or more thereof.

In particular, from the viewpoints of improving the scalp protection ability, and maintaining a good balance between an application property and a dripping property, the component (C) is preferably an acrylic acid/alkyl methacrylate copolymer, a methacrylic acid/alkyl methacrylate copolymer or a carboxyvinyl polymer, more preferably a carboxyvinyl polymer. Examples of the commercially available product of the carboxyvinyl polymer include Carbomer series, and examples thereof include Carbopol 910, Carbopol 934, Carbopol 940, Carbopol 941, Carbopol 980 and Carbopol 981 (each manufactured by Lubrizol Advanced Materials, Inc.). Examples of the commercially available product of the acrylic acid/alkyl methacrylate copolymer include Carbopol 1382, Carbopol ETD 2020, PEMULEN TR-1 and PEMULEN TR-2 (each manufactured by Lubrizol Advanced Materials, Inc.).

From the viewpoints of improving the scalp protection ability, and maintaining a good balance between an application property and a dripping property, the total content of the component (C) is, for example, 0.1 mass% or more, preferably 0.2 mass% or more, based on the total amount of the cosmetic composition. From the same viewpoints, the total content of the component (C) is, for example, 3.0 mass% or less, preferably 2.5 mass% or less, more preferably 2.0 mass% or less, further more preferably 1.7 mass% or less, even more preferably 1.5 mass% or less.

More specifically, the total content of the component (C) is, for example, 0.1 mass% or more and 3.0 mass% or less, preferably 0.1 mass% or more and 2.5 mass% or less, more preferably 0.1 mass% or more and 2.0 mass% or less, further more preferably 0.2 mass% or more and 1.7 mass% or less, even more preferably 0.2 mass% or more and 1.5 mass% or less.

(Component (D))

From the viewpoint of maintaining a good balance between an application property and a dripping property, the cosmetic composition for use in step 1 may further contain (D) a thickener different from the component (C), preferably a nonionic water-soluble polymer, or a thickener such as an ionic water-soluble polymer which does not exhibit an extreme viscosity change over the pH range of from 3 to 7. From the viewpoint of viscosity stability, the ionic water-soluble polymer is preferably in a salt form. Examples thereof include natural polymers, semisynthetic polymers, and synthetic polymers. Specific examples of the natural polymer include anionic water-soluble polymers such as xanthan gum, carrageenan and alginic acid, and nonionic water-soluble polymers such as starch. Specific examples of the semisynthetic polysaccharide-based polymer include nonionic water-soluble polymers such as hydroxy cellulose, hydroxymethyl cellulose, hydroxyethyl cellulose, hydroxypropyl cellulose and methyl cellulose, anionic water-soluble polymers such as sodium stearoxy PG-hydroxyethylcellulose sulfonate and sodium carboxymethyl cellulose, and cationic water-soluble polymers such as cationized cellulose. Specific examples of the synthetic polymer include nonionic water-soluble polymers such as polyvinyl pyrrolidone and polyvinyl alcohol, and cationic water-soluble polymers such as cationized polyvinyl pyrrolidone. These thickeners may be used singly, or in combination of two or more thereof. Examples of the commercially available product of hydroxypropyl cellulose include HPC-M and HPC-H (each manufactured by Nippon Soda Co., Ltd.), examples of the commercially available product of sodium carboxymethyl cellulose include CMC Daicel (manufactured by Daicel Chemical Industries, Ltd.), examples of the commercially available product of sodium stearoxy PG-hydroxyethylcellulose sulfonate include Poiz 310 (manufactured by Kao Corporation), and examples of the commercially available product of xanthan gum include Xanthan Gum Food Grade and Xanthan Gum CS (manufactured by Jungbunzlauer).

In particular, from the viewpoint of maintaining a good balance between an application property and a dripping property, the component (D) is preferably xanthan gum, hydroxyethyl cellulose, hydroxypropyl cellulose, sodium carboxymethyl cellulose, sodium stearoxy PG-hydroxyethylcellulose sulfonate, polyvinyl pyrrolidone or polyvinyl alcohol, more preferably sodium stearoxy PG-hydroxyethylcellulose sulfonate, xanthan gum or hydroxyethyl cellulose.

From the viewpoint of maintaining a good balance between an application property and a dripping property, the total content of the component (D) is, for example, 0.1 mass% or more, preferably 0.2 mass% or more, based on the total amount of the cosmetic composition. From the same viewpoint, the total content of the component (D) is, for example, 5.0 mass% or less, preferably 4.0 mass% or less, more preferably 3.0 mass% or less, further more preferably 2.5 mass% or less.

More specifically, from the above-described viewpoint, the total content of the component (D) is, for example, 0.1 mass% or more and 5.0 mass% or less, preferably 0.1 mass% or more and 4.0 mass% or less, more preferably 0.2 mass% or more and 3.0 mass% or less, further more preferably 0.2 mass% or more and 2.5 mass% or less.

(Aqueous medium)

For improving compatibility between the components, the cosmetic composition for use in step 1 may contain an aqueous medium in accordance with its demand. The aqueous medium refers to an organic solvent compatible with water, and examples thereof include lower alcohols such as ethanol and isopropyl alcohol; and low-molecular-weight diols and triols having 6 or less carbon atoms, such as 1,3-butylene glycol, glycerin, ethylene glycol and propylene glycol, and mixtures thereof. From the viewpoint of reducing irritation to the scalp, the content of the aqueous medium in the cosmetic composition is, for example, 10.0 mass% or less, preferably 5.0 mass% or less, more preferably 4.0 mass% or less, further more preferably 3.0 mass% or less, even more preferably 2.0 mass% or less, even more preferably 1.0 mass% or less, based on the total amount of the cosmetic composition, and it is most preferable that the cosmetic composition be substantially free of the cosmetic composition.

(Water)

The cosmetic composition for use in step 1 contains water. As the water, tap water, deionized water, distilled water and the like can be used. The content of the water in the cosmetic composition can be appropriately adjusted on the basis of the contents of other components, and is preferably 50 mass% or more, more preferably 60 mass% or more, based on the total amount of the cosmetic composition. The content of the water is preferably 99.8 mass% or less, more preferably 98 mass% or less. More specifically, the content of the water is preferably 50 mass% or more and 99.8 mass% or less, more preferably 60 mass% or more and 98 mass% or less.

(Surfactant)

Though not an essential component, a surfactant can be optionally used for improving compatibility among the components. The surfactant is not particularly limited, and examples thereof may include typical cationic surfactants, anionic surfactants, nonionic surfactants and ampholytic surfactants. Since a large content of the surfactant may cause a decrease in scalp protection ability and deterioration of a desired hair treatment effect, the total content of the surfactant is, for example, less than 5.0 mass%, preferably less than 4.0 mass%, more preferably less than 3.0 mass%, further more preferably less than 2.0 mass%, even more preferably less than 1.0 mass%, based on the total amount of the cosmetic composition, and it is most preferable that the cosmetic composition be substantially free of the surfactant.

(Other components)

In accordance with desired effects, components conventionally used for a cosmetic composition can be appropriately employed in addition to the above-described components in the cosmetic composition for use in step 1. Examples of such optional components include vitamin preparations other than the component (B); bactericides; anti-inflammatory agents such as glycyrrhizic acid and salts thereof such as dipotassium glycyrrhizinate, and glycyrrhetinic acid and salts thereof; preservatives such as benzoic acid and salts thereof; chelating agents other than the component (A); moisturizing agents such as sorbitol and panthenol; stabilizers; extracts, such as eucalyptus extract such as polar solvent extract of eucalyptus, conchiolin or hydrolysates thereof, honey, royal jerry, protein obtained from silk or hydrolysates thereof, protein-containing extract obtained from seeds of legume plants, Asian ginseng extract, rice germ extract, fucus extract, aloe extract, lotus extract, pomegranate extract, eglantine extract, chamomilla extract, glycyrrhiza extract, galangal leaf extract and chlorella extract; fragrances; ultraviolet absorbers; shea butter; rose water; sunflower oil; orange oil; and eucalyptus oil. The other components for use in the present invention are not limited thereto.

Hereinafter, the viscosity of the cosmetic composition for use in step 1 will be described.

The cosmetic composition for use in step 1 is preferably a liquid or gelled composition. In general, the pH of a composition for hair is preferably close to the pH of the scalp (from 3.5 to 6.0) for alleviating the scalp irritating sensation in application of the composition for hair. However, if the pH of an alkaline hair treatment agent (e.g. hair colorant) that is commonly used decreases, the hair treatment effect deteriorates, and thus a desired effect cannot be obtained. In particular, since the hair color composition and the hair bleach composition exhibits a hair treatment effect through an oxidation-reduction reaction with an oxidizing agent, the hair treatment effect may deteriorate in a system including an antioxidant. Thus, protecting the scalp to alleviate irritation to the scalp while maintaining the hair treatment effect is a challenge that should be overcome in this technical field.

As a result of studies, the present inventors have found that when the viscosity of the cosmetic composition for use in step 1 at pH 4.1 is in a specific range, it is possible to obtain an excellent scalp protection ability and to maintain a desired hair treatment effect as well.

The viscosity of the cosmetic composition for use in step 1 varies in accordance with the above-described contents of the components and ratios thereof, and from the viewpoints of improving the scalp protection ability and maintaining a good balance between an application property and a dripping property, the viscosity of the cosmetic composition for use in step 1 at pH 4.1 is preferably 1 mPa·s or more, more preferably 10 mPa·s or more, further more preferably 100 mPa·s or more, even more preferably 300 mPa·s or more, particularly preferably 800 mPa·s or more, more particularly preferably 1,500 mPa·s or more. From the same viewpoints, the viscosity is preferably 30,000 mPa·s or less, more preferably 25,000 mPa·s or less, further more preferably 20,000 mPa·s or less, even more preferably 15,000 mPa·s or less, particularly preferably 10,000 mPa·s or less, more particularly preferably 5,000 mPa·s or less, further more particularly preferably 2,500 mPa·s or less. The viscosity can be measured by a method described in Examples.

More specifically, from the above-described viewpoints, the viscosity is 1 mPa·s or more and 30,000 mPa·s or less, preferably 10 mPa·s or more and 30,000 mPa·s or less, more preferably 100 mPa·s or more and 30,000 mPa·s or less, further more preferably 300 mPa·s or more and 25,000 mPa·s or less, even more preferably 800 mPa·s or more and 20,000 mPa·s or less, even more preferably 800 mPa·s or more and 10,000 mPa·s or less, even more preferably 1,500 mPa·s or more and 5,000 mPa·s or less, even more preferably 1,500 mPa·s or more and 2,500 mPa·s or less.

When the viscosity of the cosmetic composition for use in step 1 at pH 5.0 is from 1.1 to 10 times as large as the viscosity of the cosmetic composition at pH 4.1, an effect is expected such that during application, the cosmetic composition has low viscosity, is thus easily spread over the scalp, and becomes viscous in an initial stage when the pH is increased by contact with a composition such as a hair colorant, a hair bleach, a perm chemical or a relaxer, so that the cosmetic composition is prevented from being dripped, and remains on the scalp to prevent an increase in scalp irritation due to mixing with a composition such as a hair colorant, and the like. From the viewpoints of further improving the scalp protection ability and maintaining a good balance between an application property and a dripping property, the viscosity of the cosmetic composition for use in step 1 at pH 5.0 is preferably 1.1 times or more and preferably 9 times or less, more preferably 7 times or less, further more preferably 5 times or less, even more preferably 3 times or less as large as the viscosity of the composition at pH 4.1. More specifically, from the above-described viewpoints, the viscosity of the cosmetic composition for use in step 1 at pH 5.0 is preferably from 1.1 to 9 times, more preferably from 1.1 to 7 times, further more preferably from 1.1 to 5 times, even more preferably from 1.1 to 3 times as large as the viscosity of the composition at pH 4.1.

The pH of the cosmetic composition for use in step 1 can be adjusted to be within a desired range by a typical acid or base as a pH adjusting agent. Examples of the pH adjusting agent include organic acids, inorganic acids, organic bases, and inorganic bases. In the present invention, a base can be preferably used from the viewpoint of maintaining a desired hair treatment effect, and specific examples thereof include inorganic alkali agents such as sodium hydroxide and potassium hydroxide; organic alkali agents such as monoethanolamine, diethanolamine, triethanolamine and aminopropanol; ammonia; ammonium chloride; and basic amino acids such as arginine. Among them, sodium hydroxide, potassium hydroxide, triethanolamine and arginine are preferably used from the viewpoint of maintaining a desired hair treatment effect.

From the viewpoint of improving the scalp protection ability to reduce damage to the scalp, the pH of the cosmetic composition for use in step 1 is, for example, 7.0 or less, preferably 6.5 or less, more preferably 6.0 or less, further more preferably 5.0 or less, even more preferably 4.8 or less. From the same viewpoint, the pH of the cosmetic composition is preferably 3.0 or more, more preferably 3.5 or more. More specifically, from the above-described viewpoint, the pH of the cosmetic composition is preferably from 3.0 to 7.0, more preferably from 3.0 to 6.5, further more preferably from 3.5 to 6.0, even more preferably from 3.5 to 5.0, even more preferably from 3.5 to 4.8. The pH can be measured by a method described in Examples.

The cosmetic composition for use in step 1 may be filled into a container specifically for a scalp protecting agent and used, or the cosmetic composition for use in step 1 may be formed as a supplying package, and provided and used together with container specifically for a scalp protecting agent.

The container specifically for a scalp protecting agent described above may be one that can be filled with the cosmetic composition for use in step 1, and examples of the container include PETG Ample Container manufactured by Pin Mao Plastic Industries Co., Ltd.

(Cosmetic composition and method for production thereof)

The present invention also includes a cosmetic composition for protecting the scalp, and a method for production thereof.

The cosmetic composition includes the following embodiments.

<Embodiment 1>

The composition may be the same as the cosmetic composition for use in the step 1, that is, one containing the following components (A) to (C) and having a viscosity of from 1 to 30,000 mPa·s at pH 4.1:

(A) a pH buffer;
(B) an antioxidant; and
(C) a pH-responsive thickener.

<Embodiment 2>

The cosmetic composition according to embodiment 2 of the present invention is a composition containing the following components (A) to (C) and having a viscosity of from 1 to 30,000 mPa·s at pH 4.1, wherein the content of a surfactant in the cosmetic composition is less than 5.0 mass%:

(A) a pH buffer;
(B) an antioxidant; and
(C) a pH-responsive thickener.

<Embodiment 3>

The cosmetic composition according to embodiment 3 of the present invention is a composition containing the following components (A) to (D) and having a viscosity of from 1 to 30,000 mPa·s at pH 4.1:

(A) a pH buffer;
(B) an antioxidant;
(C) a pH-responsive thickener; and
(D) a thickener different from the component (C).

<Embodiment 4>

The cosmetic composition according to embodiment 4 of the present invention is a composition containing the following components (A) to (D) and having a viscosity of from 1 to 30,000 mPa·s at pH 4.1:

(A) a pH buffer;
(B) an antioxidant;
(C) a pH-responsive thickener; and
(D) sodium stearoxy PG-hydroxyethylcellulose sulfonate.

<Embodiment 5>

The cosmetic composition according to embodiment 5 of the present invention is a composition containing the following components (A) to (D) and having a viscosity of from 1 to 30,000 mPa·s at pH 4.1:

(A) a pH buffer;
(B) an antioxidant;
(C) a pH-responsive thickener; and
(D) one or more selected from the group consisting of a nonionic water-soluble polymer and an ionic water-soluble polymer which does not exhibit an extreme viscosity change over a pH range of from 3 to 7.

The cosmetic composition of the present invention is not limited to the embodiments.

The scalp protection ability can be significantly improved by the cosmetic composition of the present invention. The cosmetic composition of the present invention ensures that even after application thereof to the scalp, a desired hair treatment effect does not deteriorate, and can be maintained at a good level. For example, when a hair color composition is applied, an excellent dyeing effect can be maintained. A good balance between an application property and a dripping property can be also maintained, so that convenience of use for users can be improved.

The method for production of the cosmetic composition of the present invention is not particularly limited. The cosmetic composition is produced by, for example, mixing components (A) to (C) and appropriate other components in a predetermined order.

Specifically, in the case of the embodiment 1, the component (C) is added to deionized water, the mixture is stirred to homogeneity, components (A) and (B) and appropriate other components are then added while cooling is performed, and the mixture is stirred to homogeneity, and cooled to room temperature, whereby a cosmetic composition can be obtained.

(Kit containing cosmetic composition)

The present invention also includes a kit obtained by combining the cosmetic composition with at least one or more hair treatment agent compositions selected from the group consisting of a hair color composition, a hair bleach composition, a perm composition and a relaxer composition. Among them, the hair color composition, the hair bleach composition, the perm composition and the relaxer composition are not particularly limited, and may be typical commercially available products, and examples thereof include Liese Bubble Color Series and Blaune Aroma and Shine Color Cream Series from Kao Corporation.

In the hair treatment method of the present invention, step 2 (applying a hair treatment agent composition) is carried out after step 1 (applying a cosmetic composition), the cosmetic composition for use in step 1 contains specific components (A) to (C), and has a viscosity of from 1 to 30,000 mPa·s at pH 4.1, and therefore the scalp protection ability can be significantly improved, so that damage to the scalp from the hair treatment agent composition can be reduced, or alleviated. According to a certain embodiment of the present invention, the hair treatment method of the present invention not only significantly improves the scalp protection ability, but also enables maintenance of a desired hair treatment effect and maintenance of a good balance between an application property and a dripping property of the cosmetic composition used, so that it is possible to improve convenience of use for users.

More specifically, according to the invention of the present application, the scalp is protected against damage from a hair color composition, a hair bleach composition, a perm composition or a relaxer composition, and thus an effect of effectively protecting the scalp is obtained. In a certain embodiment of the present invention, the composition for use in the present invention has a good application property, and thus a user can apply the composition to the scalp easily and uniformly. Further, since the composition has a low dripping property, an effect of protecting the scalp is obtained with an appropriate amount of the composition. In a certain embodiment of the present invention, the hair treatment method of the present invention ensures that a desired hair treatment effect does not deteriorate, and can be maintained at a good level. For example, when a hair color composition is applied, the dyeability of the hair color composition is not affected, and therefore it is possible to maintain excellent dyeability while protecting the scalp.

Regarding the embodiments described above, the present invention discloses hair treatment methods, cosmetic compositions for protecting the scalp, and kits thereof.

<1> A hair treatment method including the following step 1 and step 2, the step 2 being carried out after the step 1:

(step 1) applying to a scalp a cosmetic composition containing the following components (A), (B) and (C) and having a viscosity of from 1 to 30,000 mPa·s at pH 4.1:

(A) a pH buffer;
(B) an antioxidant; and
(C) a pH-responsive thickener; and

(step 2) applying to the hair at least one hair treatment agent composition selected from the group consisting of a hair color composition, a hair bleach composition, a perm composition and a relaxer composition.

<2> The hair treatment method according to <1>, wherein a content of the component (A) in the cosmetic composition is more than 0.15 mass% based on the total amount of the cosmetic composition.
<3> The hair treatment method according to <1> or <2>, wherein a content of a surfactant in the cosmetic composition is less than 5.0 mass%.
<4> The hair treatment method according to any one of <1> to <3>, wherein a total content of the component (A) and the component (B) in the cosmetic composition is 0.2 mass% or more and 6.0 mass% or less based on the total amount of the cosmetic composition.
<5> The hair treatment method according to any one of <1> to <4>, wherein a mass ratio of the component (A) to the component (B), ((A)/(B)), is 1.0 or more and 8.0 or less.

<6> The hair treatment method according to any one of <1> to <5>, wherein the cosmetic composition further contains (D) a thickener different from the component (C) .

<7> The hair treatment method according to <6>, wherein the component (D) is sodium stearoxy PG-hydroxyethylcellulose sulfonate.

<8> The hair treatment method according to any one of <1> to <7>, wherein a viscosity of the cosmetic composition at pH 5.0 is from 1.1 to 10 times as large as a viscosity of the cosmetic composition at pH 4.1.

<9> The hair treatment method according to any one of <1> to <8>, wherein the pH buffer has a buffering ability at least over a pH range of from 4.5 to 5.5.

<10> The hair treatment method according to any one of <1> to <9>, wherein the pH buffer exhibits a buffering ability at least over a pH range of from 4.5 to 5.5 by using an acid with a pKa (25°C) of from 4.0 to 6.5, and a salt thereof.

<11> The hair treatment method according to any one of <1> to <10>, wherein the pH buffer exhibits a buffering ability at least over a pH range of from 4.5 to 5.5 by using at least one acid selected from the group consisting of barbituric acid, succinic acid, citric acid, acetic acid, malic acid, phthalic acid and carbonic acid, and a salt thereof.

<12> The hair treatment method according to any one of <1> to <11>, wherein the pH buffer is preferably at least one selected from the group consisting of succinic acid and a salt thereof, malic acid and a salt thereof, and citric acid and a salt thereof, more preferably a pH buffer containing citric acid and a salt thereof, further more preferably a pH buffer containing citric acid and sodium citrate.

<13> The hair treatment method according to any one of <1> to <12>, wherein a total content of the component (A) (total content of a conjugate acid-base pair when the component (A) is the conjugate acid-base pair) is preferably more than 0.15 mass% and 10.0 mass% or less, more preferably 0.2 mass% or more and 9.0 mass% or less, further more preferably 0.5 mass% or more and 8.0 mass% or less, even more preferably 1.0 mass% or more and 6.0 mass% or less, even more preferably 1.0 mass% or more and 4.0 mass% or less, even more preferably 1.5 mass% or more and 4.0 mass% or less.

<14> The hair treatment method according to any one of <1> to <13>, wherein the component (B) is preferably one or more selected from the group consisting of dibutylhydroxytoluene, butylhydroxyanisole, δtocopherol, thiotaurine, thioglycolic acid, propanoic acid, tert-butylhydroquinone, nicotine acid or a derivative thereof, catechin, *Oenothera biennis* extract, β-carotene, a flavonoid compound, sulfurous acid or a salt thereof, ascorbic acid or a salt or derivative thereof, L-cysteine or a salt or derivative thereof, N-acetyl-L-cysteine or a salt or derivative thereof, and a polyphenol compound, more preferably one or more selected from the group consisting of ascorbic acid or a salt thereof, ascorbic acid glucoside, magnesium ascorbyl phosphate, erythorbic acid, sodium erythorbate, dibutylhydroxytoluene and catechin, further more preferably one or more selected from the group consisting of ascorbic acid or a salt thereof, ascorbic acid glucoside, magnesium ascorbyl phosphate, erythorbic acid and sodium erythorbate, even more preferably one or more selected from the group consisting of ascorbic acid and ascorbic acid glucoside.

<15> The hair treatment method according to any one of <1> to <14>, wherein a total content of the component (B) is preferably 0.01 mass% or more and 5.0 mass% or less, more preferably 0.1 mass% or more and 3.0 mass% or less, further more preferably 0.2 mass% or more and 1.5 mass% or less, further more preferably 0.2 mass% or more and 1.0 mass% or less, even more preferably 0.2 mass% or more and 0.7 mass% or less.

<16> The hair treatment method according to any one of <1> to <15>, wherein the total content of the component (A) and the component (B) is preferably 0.2 mass% or more and 10.0 mass% or less, more preferably 1.0 mass% or more and 8.0 mass% or less, further more preferably 2.3 mass% or more and 6.0 mass% or less, even more preferably 2.3 mass% or more and 5.5 mass% or less, even more preferably 2.5 mass% or more and 5.0 mass% or less, even more preferably 2.5 mass% or more and 4.5 mass% or less, even more preferably 2.5 mass% or more and 4.0 mass% or less.

<17> The hair treatment method according to any one of <1> to <16>, wherein the mass ratio of the component (A) to the component (B) [(component (A))/(component (B))] is preferably 1.0 or more and 8.0 or less, more preferably 1.1 or more and 7.5 or less, further more preferably 1.5 or more and 6.5 or less, even more preferably 2.0 or more and 6.0 or less, even more preferably 3.0 or more and 5.0 or less.

<18> The hair treatment method according to any one of <1> to <17>, wherein the pH-responsive thickener is an ionic water-soluble polymer whose viscosity increases in response to an increase in pH over a part or the whole of the pH range of from 3 to 7.

<19> The hair treatment method according to any one of <1> to <18>, wherein the pH-responsive thickener is an ionic water-soluble polymer whose viscosity increases in response to an increase in pH over a part or the whole of the pH range of from 3 to 7, the ionic water-soluble polymer containing at least one selected from the group consisting of an unneutralized carboxyl group and an unneutralized sulfonate group.

<20> The hair treatment method according to any one of <1> to <19>, wherein the pH-responsive thickener is a linear or branched polymer having one or more selected from the group consisting of acrylic acid, methacrylic acid, maleic acid, fumaric acid, itaconic acid, styrenesulfonic acid, vinylbenzoic acid, 2-acrylamido-2-methyl-1-propanesulfonic acid and dimethylolpropionic acid, or a crosslinked polymer or copolymer thereof.

<21> The hair treatment method according to any one of <1> to <20>, wherein the pH-responsive thickener is preferably one or more selected from the group consisting of polyacrylic acid, a carboxyvinyl polymer, sodium polyacrylate, an acrylic acid/alkyl methacrylate copolymer, polymethacrylic acid and a methacrylic acid/alkyl methacrylate copolymer, more preferably one or more selected from the group consisting of an acrylic acid/alkyl methacrylate copolymer, a methacrylic acid/alkyl methacrylate copolymer and a carboxyvinyl polymer, further more preferably one containing a carboxyvinyl polymer.

<22> The hair treatment method according to any one of <1> to <21>, wherein a total content of the component (C) is preferably 0.1 mass% or more and 3.0 mass% or less, more preferably 0.1 mass% or more and 2.5 mass% or less, further more preferably 0.1 mass% or more and 2.0 mass% or less, even more preferably 0.2 mass% or more and 1.7 mass% or less, even more preferably 0.2 mass% or more and 1.5 mass% or less.

<23> The hair treatment method according to any one of <1> to <22>, wherein the cosmetic composition further contains, as the (D) thickener, one or more selected from the group consisting of a nonionic water-soluble polymer and an ionic water-soluble polymer which does not exhibit an extreme viscosity change over a pH range of from 3 to 7.

<24> The hair treatment method according to any one of <6> to <23>, wherein the component (D) is preferably one containing one or more selected from the group consisting of xanthan gum, carrageenan, alginic acid, starch, hydroxy cellulose, hydroxymethyl cellulose, hydroxyethyl cellulose, hydroxypropyl cellulose, sodium stearoxy PG-hydroxyethylcellulose sulfonate, sodium carboxymethyl cellulose, methyl cellulose, cationized cellulose, polyvinyl pyrrolidone, polyvinyl alcohol and cationized polyvinyl pyrrolidone, more preferably one containing one or more selected from the group consisting of xanthan gum, hydroxyethyl cellulose, hydroxypropyl cellulose, sodium carboxymethyl cellulose, sodium stearoxy PG-hydroxyethylcellulose sulfonate, polyvinyl pyrrolidone and polyvinyl alcohol, further more preferably one containing one or more selected from the group consisting of sodium stearoxy PG-hydroxyethylcellulose sulfonate, xanthan gum and hydroxyethyl cellulose.

<25> The hair treatment method according to any one of <6> to <24>, wherein the total content of the component (D) is preferably 0.1 mass% or more and 5.0 mass% or less, more preferably 0.1 mass% or more and 4.0 mass% or less, further more preferably 0.2 mass% or more and 3.0 mass% or less, even more preferably 0.2 mass% or more and 2.5 mass% or less.

<26> The hair treatment method according to any one of <1> to <25>, wherein the total content of a water-soluble solvent in the cosmetic composition is preferably 10.0 mass% or less, more preferably 5.0 mass% or less, further more preferably 4.0 mass% or less, even more preferably 3.0 mass% or less, even more preferably 2.0 mass% or less, even more preferably 1.0 mass% or less, where the cosmetic composition is substantially free of the water-soluble solvent.

<27> The hair treatment method according to any one of <1> to <26>, wherein the content of water in the cosmetic composition is preferably 50 mass% or more and 99.8 mass% or less, more preferably 60 mass% or more and 98 mass% or less.

<28> The hair treatment method according to any one of <1> to <27>, wherein the total content of the surfactant in the cosmetic composition is preferably less than 5.0 mass%, more preferably less than 4.0 mass%, further more preferably less than 3.0 mass%, even more preferably less than 2.0 mass%, even more preferably less than 1.0 mass%, and it is even more preferably that the cosmetic composition be substantially free of the surfactant.

<29> The hair treatment method according to any one of <1> to <28>, wherein the viscosity of the cosmetic composition at pH 4.1 is preferably 10 mPa·s or more and 30,000 mPa·s or less, more preferably 100 mPa·s or more and 30,000 mPa·s or less, further more preferably 300 mPa·s or more and 25,000 mPa·s or less, even more preferably 800 mPa·s or more and 20,000 mPa·s or less, even more preferably 800 mPa·s or more and 10,000 mPa·s or less, even more preferably 1,500 mPa·s or more and 5,000 mPa·s or less, even more preferably 1,500 mPa·s or more and 2,500 mPa·s or less.

<30> The hair treatment method according to any one of <1> to <29>, wherein the viscosity of the cosmetic composition at pH 5.0 is preferably 1.1 to 9 times, more preferably 1.1 to 7 times, further more preferably 1.1 to 5 times, even more preferably 1.1 to 3 times as large as the viscosity of the cosmetic composition at pH 4.1.

<31> The hair treatment method according to any one of <1> to <30>, wherein the cosmetic composition further contains a pH adjusting agent, and the pH adjusting agent is preferably one or more selected from the group consisting of sodium hydroxide, potassium hydroxide, monoethanolamine, diethanolamine, triethanolamine, aminopropanol, ammonia, ammonium chloride and arginine, more preferably one or more selected from the group consisting of sodium hydroxide, potassium hydroxide, triethanolamine and arginine.

<32> The hair treatment method according to any one of <1> to <31>, wherein the cosmetic composition has pH of preferably from 3.0 to 7.0, more preferably from 3.0 to 6.5, further more preferably from 3.5 to 6.0, even more preferably from 3.5 to 5.0, even more preferably from 3.5 to 4.8.

<33> The hair treatment method according to any one of <1> to <32>, wherein in the step 1, an amount of the cosmetic composition applied to the scalp is preferably in the range of from 8 to 40 $\mu$L/cm$^2$, more preferably in the range of 13 to 27 $\mu$L/cm$^2$, further more preferably in the range of 20 to 27 $\mu$L/cm$^2$.

<34> A cosmetic composition for protecting the scalp, containing the following components (A), (B) and (C) and having a viscosity of from 1 to 30,000 mPa·s at pH 4.1:

(A) a pH buffer;
(B) an antioxidant; and
(C) a pH-responsive thickener.

<35> The cosmetic composition according to <34>, wherein the pH buffer has a buffering ability at least over a pH range of from 4.5 to 5.5.

<36> A cosmetic composition for protecting the scalp, containing the following components (A), (B) and (C), wherein

the component (A) exhibits a buffering ability at least over a pH range of from 4.5 to 5.5 by using an acid with a pKa (25°C) of from 4.0 to 6.5, and a salt thereof,
the component (B) is one or more selected from the group consisting of ascorbic acid or a salt thereof, ascorbic acid glucoside, magnesium ascorbyl phosphate, erythorbic acid, and sodium erythorbate,
the component (C) is an ionic water-soluble polymer whose viscosity increases in response to an increase in pH over a part or the whole of the pH range of from 3 to 7, the ionic water-soluble polymer containing at least one selected from the group consisting of an unneutralized carboxyl group and an unneutralized sulfonate group,
the cosmetic composition has pH of from 3.5 to 4.8, and
the cosmetic composition has a viscosity at pH 4.1 of from 100 to 30,000 mPa·s.

<37> A cosmetic composition for protecting the scalp, containing the following components (A), (B) and (C), wherein

the component (A) exhibits a buffering ability at least over a pH range of from 4.5 to 5.5 by using an acid with a pKa (25°C) of from 4.0 to 6.5, and a salt thereof,
the component (B) is one or more selected from the group consisting of ascorbic acid or a salt thereof, ascorbic acid glucoside, magnesium ascorbyl phosphate, erythorbic acid, and sodium erythorbate,
the component (C) is an ionic water-soluble polymer whose viscosity increases in response to an increase in pH over a part or the whole of the pH range of from 3 to 7, the ionic water-soluble polymer containing at least one selected from the group consisting of an unneutralized carboxyl group and an unneutralized sulfonate group,
the cosmetic composition has pH of from 3.5 to 4.8, a content of a surfactant is less than 1.0 mass%, and
the cosmetic composition has a viscosity at pH 4.1 of from 100 to 30,000 mPa·s.

<38> The cosmetic composition according to any one of <34> to <36>, wherein the content of the component (A) in the cosmetic composition is more than 0.15 mass% and the content of the surfactant is less than 5.0 mass%, based on the total amount of the cosmetic composition.

<39> The cosmetic composition according to any one of <34> to <36>, wherein the content of the component (A) in the cosmetic composition is more than 0.15 mass%, the total content of the component (A) and the component (B) in the cosmetic composition is 2.3 mass% or more and 6.0 mass% or less and the content of the surfactant is less than 5.0 mass%, based on the total amount of the cosmetic composition.

<40> A kit containing:

(I) a cosmetic composition containing the following components (A), (B) and (C) and having a viscosity of from 1 to 30,000 mPa·s at pH 4.1:

(A) a pH buffer;
(B) an antioxidant; and
(C) a pH-responsive thickener; and

(II) at least one or more hair treatment agent compositions selected from the group consisting of a hair color composition, a hair bleach composition, a perm composition and a relaxer composition.

<41> The kit according to <40>, wherein the pH buffer has a buffering ability at least over a pH range of from 4.5 to 5.5.

Examples

Hereinafter, the present invention will be described by way of Examples, which should not be construed as limiting the present invention. In Examples, various measurements and evaluations were performed by the following methods.

(pH measurement)

The pH of an undiluted solution was measured at 25°C using a pH meter (pH METER F-52 manufactured by HORIBA, Ltd.).

(Viscosity measurement)

In the present invention, the viscosity is a value obtained by performing measurement using a Brookfield viscometer (VISCOMETER TVB-10 manufactured by TOKI SANGYO CO., LTD.), followed by rotation for 10 seconds. For the rotor used in the measurement and the rotation speed, appropriate conditions are selected depending on the viscosity of the cosmetic composition and in accordance with a manual for a measuring machine, and the like. Examples of the conditions are:

less than 200 mPa·s = Rotor No. 1, 30 rpm;
200 mPa·s or more and less than 4,000 mPa·s = Rotor No. 3, 30 rpm;
4,000 mPa·s or more and less than 20,000 mPa·s = Rotor No. 4, 30 rpm; and
20,000 mPa·s or more and less than 50,000 mPa·s = Rotor No. 4, 12 rpm.

(Evaluation of scalp protection ability)

Pig hide (Funakoshi Co., Ltd.) was washed with shampoo (as shown in Table 1 below), and then sufficiently dried, and 20 μL of a cosmetic composition sample (hereinafter, referred to as a "sample") was spread over a 1.5-centimeter square area of the pig hide. Onto this, 100 μL of a hair bleach was further applied (as shown in Table 2; a first part and a second part were mixed at a ratio of 1 : 1 at the time of evaluation), left to stand at room temperature for 30 minutes, and then washed off with water at about 40°C for 30 seconds, and the pig hide was dried. After the pig hide was sufficiently dried, a 1.5-centimeter square tape (acrylic tape manufactured by TERAOKA SEISAKUSHO CO., LTD.) was bonded to the sample-treated portion, and tape was stripped three times, so that horny layers were collected on the sticky surfaces of the tapes. Each tape was cut into four pieces, and put in a vial container, and 450 μL of a mixed aqueous solution of 0.1 N NaOH and 1% sodium dodecyl sulfate was added, and protein was extracted in an incubator at 60°C for 2.5 hours. The extract was cooled with water, and 50 μL of 2 N HCl was added to prepare a horny layer protein extraction sample.

The amount of horny layer protein was determined using BCA Protein Assay Kit (manufactured by Thermo Fisher Scientific). 20 μL of an attached standard solution and 20 μL of the horny layer protein extraction sample were put in a 96-well plate, 200 μL of an attached color developing liquid (mixed at a ratio of A : B = 50 : 1) was further added, and the mixture was warmed in an incubator at 37°C for 30 minutes. The absorbance at 562 nm was measured with a microplate reader (Sunrise Rainbow Thermo: RC-R manufactured by Tecan Ltd.), and the amount of horny layer protein in the extraction liquid was calculated from a calibration curve of the standard solution. The scalp protection ability was calculated from the following equation (1), where the larger the value, the higher the scalp protection ability.

$$\text{Scalp protection ability (\%)} = \left( 1 - \frac{\text{Amount of protein after treatment of each sample}}{\text{Amount of protein without treatment with sample}} \right) \times 100 \quad (1)$$

[Table 1]

| Shampoo formulation (pH 7) | Active amount (mass%) |
|---|---|
| Sodium edetate | 0.3 |
| Sodium benzoate | 0.5 |
| Lauramide DEA (diethanolamide)*1 | 1.5 |
| Sodium laureth sulfate*2 | 15.5 |

(continued)

| Shampoo formulation (pH 7) | Active amount (mass%) |
|---|---|
| Phosphoric acid | q.s. |
| Water | Balance |
| Total | 100 |

*1: Aminon L-02 manufactured by Kao Corporation
*2: Sodium Polyoxyethylene Alkyl Sulfate manufactured by Kao Corporation, in which the average number of carbon atoms in the alkyl chain is 12 and the average addition molar number of EO is 2

[Table 2]

| Hair bleach formulation | Active amount (mass%) | |
|---|---|---|
| | First part | Second part |
| Hydrogen peroxide (aqueous solution) | -- | 7.6 |
| Ammonia (aqueous solution) | 6.00 | -- |
| Ammonium chloride | 0.86 | -- |
| Monoethanolamine | 0.84 | -- |
| Water | Balance | Balance |

(Evaluation of dyeability)

350 mg of the sample was spread over the entirety of 1 g of Chinese Gray Hair Tress (manufactured by Beaulax). Immediately thereafter, a hair colorant (Blaune Bubble Color 4: Light Brown; manufactured by Kao Corporation) was applied to a tress of 1 g of gray hair at a bath ratio of 0.7, and left to stand at 30°C for 20 minutes, and the hair was then rinsed with water at about 40°C, washed with the shampoo in Table 1 above, and washed with water. Subsequently, hair rinse (as shown in Table 3 below) was applied, and the hair was then rinsed with water at about 40°C, wiped with a towel, and dried with a drier. The color of the obtained hair tress immediately after dyeing of the hair was measured in the CIE color coordinate system (L*, a*, b*) using a color-difference meter (Chroma Meter CR-400 manufactured by Konica Minolta Sensing Inc.), and a difference in color between the hair tress and a hair tress dyed without treatment with the sample was determined from the following expression (2), and defined as dyeability $\Delta E^*$. $L^*_0$, $a^*_0$ and $b^*_0$ represent the values of L*, a* and b*, respectively, for the hair tress dyed without treatment with the sample, and $L^*_1$, $a^*_1$ and $b^*_1$ represent the values of L*, a* and b*, respectively, for the hair tress dyed immediately after treatment with the sample. A smaller value of the $\Delta E^*$ indicates that the sample is less influenced on dyeability.

$$\Delta E^* = \sqrt{(L_1^* - L_0^*)^2 + (a_1^* - a_0^*)^2 + (b_1^* - b_0^*)^2}$$

$$(2)$$

[Table 3]

| Rinse formulation (pH 7) | Active amount (mass%) |
|---|---|
| Methylparaben | 0.1 |
| Cetearyl alcohol | 2.0 |
| Propylene glycol | 5.0 |
| Distearyldimonium chloride*3 | 2.7 |
| Stearyltrimonium chloride*4 | 0.8 |

(continued)

| Rinse formulation (pH 7) | Active amount (mass%) |
|---|---|
| Water | Balance |
| Total | 100 |
| *3: Quartamin D86P manufactured by Kao Corporation<br>*4: Quartamin 86W manufactured by Kao Corporation | |

(Evaluation of application property)

20 mL of the sample was put in a PETG ampule container (manufactured by Pin Mao Plastic Industries Co., Ltd.; volume: about 20 mL), and from the container, 0.72 g of the sample was applied to a scalp sample having hair over a 6-centimeter square area (the scalp sample has a rubber tress manufactured by Beaulax), and spread by rubbing with a finger for 10 seconds. The spreadability is evaluated on a six-grade scale.

    6. Very easy to spread
    5. Easy to spread
    4. Slightly easy to spread
    3. Fair
    2. Slightly difficult to spread
    1. Difficult to spread

(Evaluation of dripping property)

0.2 mL of a liquid droplet (diameter: 1 cm) of each sample was dropped to a sheet made of polypropylene, and the sheet was inclined by 90 degrees. After 30 seconds, the distance over which the liquid extended was measured.

(Examples 1 to 43 and Comparative Examples 1 to 4)

The cosmetic compositions of Examples were obtained by performing preparation on the basis of the compositions shown in Tables 4 to 9, and were evaluated in accordance with the methods described above. The numerical values related to components such as components (A), (B), (C) and (D) in Tables 4 to 9 represent active amounts of the respective components in the respective compositions which are expressed in mass%.

[Table 4]

| | Component name | Example 1 | Comparative Example 1 | Comparative Example 2 | Comparative Example 3 | Example 2 | Example 3 | Example 4 | Comparative Example 4 | Example 5 |
|---|---|---|---|---|---|---|---|---|---|---|
| (A) pH Buffer | Citric acid*5 | 0.8 | -- | 0.8 | 0.8 | 0.8 | 0.8 | 0.8 | 0.8 | 0.8 |
| | Sodium citrate*6 | 1.2 | -- | 1.2 | 1.2 | 1.2 | 1.2 | 1.2 | 1.2 | 1.2 |
| (B) Antioxidant | Ascorbic acid glucoside*7 | 0.50 | 0.50 | - | 0.50 | 0.50 | 0.50 | 0.50 | 0.50 | 0.50 |
| (C) pH-responsive thickener | Carbomer (Carbopol 981)*8 | 1.35 | 1.35 | 1.35 | -- | 0.60 | 0.50 | 1.00 | 1.60 | 0.40 |
| (D) Other thickeners | Sodium stearoxy PG-hydroxyethylcellulose sulfonate *9 | -- | -- | -- | -- | -- | -- | -- | -- | 0.36 |
| | Xanthan gum*10 | -- | -- | -- | -- | -- | 0.50 | 1.40 | 2.40 | -- |
| | 48% sodium hydroxide | q.s. | q.s. | q.s. | q.s. | q.s. | q.s. | q.s. | q.s. | q.s. |
| | Water | Balance | Balance | Balance | Balance | Balance | Balance | Balance | Balance | Balance |
| pH (after adjustment) | | 4.1 | 4.1 | 4.1 | 4.1 | 4.1 | 4.1 | 4.1 | 4.1 | 4.1 |
| Content of (A) (mass%) | | 2.0 | - | 2.0 | 2.0 | 2.0 | 2.0 | 2.0 | 2.0 | 2.0 |
| Total content of (A) + (B) (mass%) | | 2.5 | 0.5 | 2.0 | 2.5 | 2.5 | 2.5 | 2.5 | 2.5 | 2.5 |
| Mass ratio of ((A)/(B)) | | 4.0 | -- | -- | 4.0 | 4.0 | 4.0 | 4.0 | 4.0 | 4.0 |
| Viscosity at pH 4.1 (mPa-s) | | 2080 | 7330 | 2740 | 1 | 79 | 2188 | 24370 | 42710 | 1713 |
| Viscosity at pH 5.0 (mPa·s) | | 5100 | 9680 | 5420 | 1 | 620 | 2995 | 28960 | 37010 | 1604 |
| Viscosity change from viscosity at pH 4.1 to viscosity at pH 5.0 (times) | | 2.5 | 1.3 | 2.0 | 1.0 | 7.8 | 1.4 | 1.2 | 0.9 | 0.9 |

(continued)

| Evaluation | Component name | Example 1 | Comparative Example 1 | Comparative Example 2 | Comparative Example 3 | Example 2 | Example 3 | Example 4 | Comparative Example 4 | Example 5 |
|---|---|---|---|---|---|---|---|---|---|---|
| | Scalp protection ability (%) | 36.6 | 6.9 | 4.8 | 7.6 | 25.3 | 31.1 | 26.6 | 7.0 | 31.7 |
| | Dyeability ($\Delta E^*$) | 0.87 | 2.07 | 1.64 | 0.85 | 1.72 | 0.45 | 0.94 | 0.88 | 0.64 |
| | Application property | 5 | 4 | 4 | 2 | 3 | 5 | 3 | 1 | 6 |
| | Dripping property (cm) | 2.1 | 0.2 | 1.0 | >30 | >30 | 1.7 | 0.0 | 0.0 | 2.8 |

*5: Citric Acid-Hydrate manufactured by San Fu Chemical Co., Ltd.

*6: Trisodium Citrate manufactured by Iwata Chemical Co., Ltd.

*7: Ascorbic acid 2-Glucoside manufactured by HAYASHIBARA CO., LTD.

*8: Carbopol 981 manufactured by Lubrizol Advanced Materials, Inc.

*9: Poiz 310 manufactured by Kao Corporation *10: Xanaxthan Gum Food Grade manufactured by Jungbunzlauer Austria AG

[Table 5]

| | Component name | Example 6 | Example 7 | Example 8 | Example 9 | Example 10 | Example 11 | Example 12 | Example 13 | Example 14 |
|---|---|---|---|---|---|---|---|---|---|---|
| (A) pH buffer | Citric acid*5 | 0.80 | 0.80 | 0.80 | 0.80 | 0.80 | 0.80 | 0.08 | 0.80 | 0.64 |
| | Sodium citrate | 1.20 | 1.20 | 1.20 | 1.20 | 1.20 | 1.20 | 0.12 | 1.20 | 0.96 |
| (B) Antioxidant | Ascorbic acid glucoside | 0.50 | 0.50 | 0.50 | 0.50 | 0.50 | 0.50 | 0.50 | 0.50 | 0.40 |
| (C) pH-responsive thickener | Carbomer (Carbopol 981) | 0.80 | 1.00 | 1.30 | 1.50 | 1.00 | 1.00 | 0.50 | 0.50 | 0.50 |
| (D) Other thickeners | Sodium stearoxy PG-hydroxyethylcellulose sulfonate | -- | -- | -- | -- | -- | -- | -- | -- | -- |
| | Xanthan gum | -- | --- | -- | -- | 0.24 | 1.20 | 0.5 | 0.5 | 0.5 |
| | Surfactant* 11 | -- | -- | -- | -- | -- | -- | -- | 4.5 | -- |
| | 48% sodium hydroxide | q.s. | q.s. | q.s. | q.s. | q.s. | q.s. | q.s. | q.s. | q.s. |
| | Water | Balance | Balance | Balance | Balance | Balance | Balance | Balance | Balance | Balance |
| pH (after adjustment) | | 4.1 | 4.1 | 4.1 | 4.1 | 4.1 | 4.1 | 4.1 | 4.1 | 4.1 |
| Content of (A) (mass%) | | 2.0 | 2.0 | 2.0 | 2.0 | 2.0 | 2.0 | 0.20 | 2.0 | 1.60 |
| Total content of (A) + (B) (mass%) | | 2.5 | 2.5 | 2.5 | 2.5 | 2.5 | 2.5 | 0.7 | 2.5 | 2.0 |
| Mass ratio of ((A)/(B)) | | 4.0 | 4.0 | 4.0 | 4.0 | 4.0 | 4.0 | 0.4 | 4.0 | 4.0 |
| Viscosity at pH 4.1 (mPa-s) | | 381 | 792 | 1712 | 2787 | 4950 | 9330 | 3143 | 2216 | 2276 |
| Viscosity at pH 5.0 (mPa·s) | | 1273 | 2272 | 4880 | 6360 | 7160 | 15270 | 5400 | 2747 | 3231 |
| Viscosity change from viscosity at pH 4.1 to viscosity at pH 5.0 (times) | | 3.3 | 2.9 | 2.9 | 2.3 | 1.4 | 1.6 | 1.7 | 1.2 | 1.4 |
| Evaluation | Scalp protection ability (%) | 25.6 | 25.2 | 31.0 | 30.5 | 29.1 | 28.7 | 14.3 | 13.8 | 20.5 |
| | Dyeability (ΔE*) | 0.74 | 0.65 | 1.28 | 0.79 | 0.92 | 1.24 | 0.79 | 1.85 | 0.34 |
| | Application property | 3 | 3 | 5 | 4 | 4 | 3 | 4 | 5 | 5 |
| | Dripping property (cm) | 18.1 | 9.0 | 3.0 | 1.3 | 0.0 | 0.0 | 0.6 | 1.7 | 1.5 |

*11: Emanon CH-60 (K) manufactured by Kao Corporation

[Table 6]

| | Component name | Example 15 | Example 16 | Example 17 | Example 18 | Example 19 | Example 20 | Example 21 | Example 22 | Example 23 |
|---|---|---|---|---|---|---|---|---|---|---|
| (A) pH buffer | Citric acid*5 | 0.96 | 1.28 | 1.76 | 2.24 | 0.33 | 0.60 | 0.88 | 0.89 | 0.80 |
| | Sodium citrate | 1.44 | 1.92 | 2.64 | 3.36 | 0.50 | 0.90 | 1.32 | 1.34 | 1.20 |
| (B) Antioxidant | Ascorbic acid glucoside | 0.60 | 0.80 | 1.10 | 1.40 | 1.67 | 1.00 | 0.29 | 0.26 | 0.50 |
| (C) pH-responsive thickener | Carbomer (Carbopol 981) | 0.50 | 0.50 | 0.50 | 0.50 | 0.50 | 0.50 | 0.50 | 0.50 | 0.50 |
| (D) Other thickeners | Sodium stearoxy PG-hydroxyethylcellulose sulfonate | -- | -- | -- | -- | -- | -- | -- | -- | -- |
| | Xanthan gum | 0.5 | 0.5 | 0.5 | 0.5 | 0.5 | 0.5 | 0.5 | 0.5 | -- |
| | Hydroxyethyl cellulose*12 | -- | -- | -- | -- | -- | -- | -- | -- | 0.65 |
| | 48% sodium hydroxide | q.s. | q.s. | q.s. | q.s. | q.s. | q.s. | q.s. | q.s. | q.s. |
| | Water | Balance | Balance | Balance | Balance | Balance | Balance | Balance | Balance | Balance |
| pH (after adjustment) | | 4.1 | 4.1 | 4.1 | 4.1 | 4.1 | 4.1 | 4.1 | 4.1 | 4.1 |
| Content of (A) (mass%) | | 2.40 | 3.20 | 4.40 | 5.60 | 0.83 | 1.50 | 2.21 | 2.24 | 2.00 |
| Total content of (A) + (B) (mass%) | | 3.0 | 4.0 | 5.5 | 7.0 | 2.5 | 2.5 | 2.5 | 2.5 | 2.5 |
| Mass ratio of ((A)/(B)) | | 4.0 | 4.0 | 4.0 | 4.0 | 0.5 | 1.5 | 7.5 | 8.5 | 4.0 |
| Viscosity at pH 4.1 (mPa-s) | | 2024 | 2028 | 1792 | 1848 | 2180 | 2156 | 2108 | 2116 | 1947 |
| Viscosity at pH 5.0 (mPa·s) | | 2915 | 2659 | 2463 | 2383 | 3287 | 3211 | 3103 | 2935 | 2954 |
| Viscosity change from viscosity at pH 4.1 to viscosity at pH 5.0 (times) | | 1.4 | 1.3 | 1.4 | 1.3 | 1.5 | 1.5 | 1.5 | 1.4 | 1.5 |
| Evaluation | Scalp protection ability (%) | 28.0 | 25.8 | 27.2 | 31.5 | 19.8 | 24.4 | 23.6 | 20.4 | 20.7 |
| | Dyeability ($\Delta$E*) | 1.35 | 1.36 | 2.01 | 2.88 | 0.67 | 0.67 | 1.58 | 2.12 | 1.45 |
| | Application property | 5 | 5 | 5 | 5 | 5 | 5 | 5 | 5 | 5 |
| | Dripping property (cm) | 1.9 | 2.2 | 2.4 | 2.9 | 1.2 | 1.7 | 2 | 2 | 5.3 |

*12: HEC Daicel SE850 manufactured by Daicel Miraizu Ltd.

[Table 7]

| | Component name | Example 24 | Example 25 | Example 26 | Example 27 | Example 28 | Example 29 |
|---|---|---|---|---|---|---|---|
| (A) pH buffer | Citric acid | 0.80 | 0.80 | 0.80 | 0.80 | 0.80 | 0.80 |
| | Sodium citrate | 1.20 | 1.20 | 1.20 | 1.20 | 1.20 | 1.20 |
| (B) Antioxidant | Ascorbic acid glucoside | 0.50 | 0.50 | 0.50 | 0.50 | 0.50 | 0.50 |
| (C) pH-responsive thickener | Carbomer (Carbopol 981) | 1.00 | 0.08 | 0.20 | 0.80 | 1.20 | 1.35 |
| (D) Other thickeners | Sodium stearoxy PG-hydroxyethylcellulose sulfonate | 0.20 | -- | -- | -- | -- | -- |
| | Xanthan gum | -- | 0.85 | 0.7 | 0.29 | 0.14 | 0.02 |
| | 48% sodium hydroxide | q.s. | q.s. | q.s. | q.s. | q.s. | q.s. |
| | Water | Balance | Balance | Balance | Balance | Balance | Balance |
| pH (after adjustment) | | 4.1 | 4.1 | 4.1 | 4.1 | 4.1 | 4.1 |
| Content of (A) (mass%) | | 2.00 | 2.00 | 2.00 | 2.00 | 2.00 | 2.00 |
| Total content of (A) + (B) (mass%) | | 2.5 | 2.5 | 2.5 | 2.5 | 2.5 | 2.5 |
| Mass ratio of ((A)/(B)) | | 4.0 | 4.0 | 4.0 | 4.0 | 4.0 | 4.0 |
| Viscosity at pH 4.1 (mPa·s) | | 1997 | 2220 | 2024 | 2102 | 2184 | 1709 |
| Viscosity at pH 5.0 (mPa·s) | | 3159 | 2074 | 2240 | 4320 | 6030 | 5370 |
| Viscosity change from viscosity at pH 4.1 to viscosity at pH 5.0 (times) | | 1.6 | 0.9 | 1.1 | 2.1 | 2.8 | 3.1 |
| Evaluation | Scalp protection ability (%) | 30.8 | 14.6 | 27.1 | 29.8 | 28.6 | 29.5 |
| | Dyeability (ΔE*) | 0.87 | 1.10 | 0.64 | 0.90 | 1.56 | 2.19 |
| | Application property | 5 | 5 | 5 | 5 | 5 | 5 |
| | Dripping property (cm) | 3.5 | 5.1 | 2.6 | 2.3 | 3 | 4.8 |

[Table 8]

| | Component name | Example 30 | Example 31 | Example 32 | Example 33 | Example 34 | Example 35 | Example 36 | Example 37 |
|---|---|---|---|---|---|---|---|---|---|
| (A) pH buffer | Citric acid | -- | 0.80 | 0.60 | 0.32 | 0.80 | 0.80 | 0.80 | 0.80 |
| | Sodium citrate | -- | 1.20 | 0.10 | 0.48 | 1.20 | 1.20 | 1.20 | 1.20 |
| | Succinic acid*13 | 0.80 | -- | -- | -- | -- | -- | -- | -- |
| | Disodium succinate*14 | 1.20 | -- | -- | -- | -- | -- | -- | -- |
| (B) Antioxidant | Ascorbic acid glucoside | 0.50 | 0.50 | 0.04 | 0.20 | 0.50 | 0.50 | 0.50 | 0.50 |
| (C) pH-responsive thickener | Carbomer (Carbopol 981) | 0.50 | -- | 0.50 | 0.50 | 0.50 | 0.50 | 0.50 | 0.50 |
| | Pemulen TR1*15 | -- | 0.50 | -- | -- | -- | -- | -- | -- |
| (D) Other thickeners | Sodium stearoxy PG-hydroxyethylcellulose sulfonate | -- | -- | -- | -- | -- | -- | -- | -- |
| | Xanthan gum | 0.50 | 0.50 | 0.50 | 0.50 | 0.50 | 0.50 | 0.50 | 0.50 |
| | 48% sodium hydroxide | q.s. | q.s. | q.s. | q.s. | q.s. | q.s. | q.s. | q.s. |
| | Water | Balance | Balance | Balance | Balance | Balance | Balance | Balance | Balance |
| pH (after adjustment) | | 4.1 | 4.1 | 4.1 | 4.1 | 5.0 | 6.0 | 6.5 | 7.0 |
| Content of (A) (mass%) | | 2.00 | 2.00 | 0.20 | 1.00 | 2.00 | 2.00 | 2.00 | 2.00 |
| Total content of (A) + (B) (mass%) | | 2.5 | 2.5 | 0.2 | 1.0 | 2.5 | 2.5 | 2.5 | 2.5 |
| Mass ratio of ((A)/(B)) | | 4.0 | 4.0 | 4.0 | 4.0 | 4.0 | 4.0 | 4.0 | 4.0 |
| Viscosity at pH 4.1 (mPa·s) | | 2280 | 1633 | 3233 | 2700 | 2188 | 2188 | 2188 | 2188 |
| Viscosity at pH 5.0 (mPa·s) | | 2793 | 2469 | 5670 | 3770 | 2995 | 2995 | 2995 | 2995 |
| Viscosity change from viscosity at pH 4.1 to viscosity at pH 5.0 (times) | | 1.2 | 1.5 | 1.8 | 1.4 | 1.4 | 1.4 | 1.4 | 1.4 |

EP 4 190 306 A1

22

(continued)

| Evaluation | Component name | Example 30 | Example 31 | Example 32 | Example 33 | Example 34 | Example 35 | Example 36 | Example 37 |
|---|---|---|---|---|---|---|---|---|---|
| | Scalp protection ability (%) | 29.0 | 30.1 | 15.6 | 19.1 | 21.6 | 22.1 | 17.0 | 13.7 |
| | Dyeability ($\Delta E^*$) | 2.91 | 1.56 | 0.53 | 0.66 | 0.51 | 0.45 | 0.41 | 0.49 |
| | Application property | 5 | 5 | 5 | 5 | 5 | 5 | 5 | 5 |
| | Dripping property (cm) | 1.7 | 2.1 | 0.8 | 1.3 | 0.6 | 0.6 | 0.6 | 0.5 |

*13: Succinic Acid manufactured by Nippon Shokubai Co., Ltd.

*14: Disodium Succinate manufactured by Fuso Chemical Co., Ltd.

*15: Pemulen TR-1 manufactured by Lubrizol Advanced Materials, Inc.

[Table 9]

| | Component name | Example 38 | Example 39 | Example 40 | Example 41 | Example 42 | Example 43 |
|---|---|---|---|---|---|---|---|
| (A) pH buffer | Citric acid | 0.80 | 0.80 | 0.80 | 0.80 | 0.80 | 0.80 |
| | Sodium citrate | 1.20 | 1.20 | 1.20 | 1.20 | 1.20 | 1.20 |
| (B) Antioxidant | Ascorbic acid[16] | 1.00 | -- | -- | -- | -- | -- |
| | Dibutylhydroxytoluene[17] | -- | 0.63 | -- | -- | -- | -- |
| | Tocopherol nicotinic acid ester[18] | -- | -- | 1.00 | -- | -- | -- |
| | Sodium sulfite[19] | -- | -- | -- | 0.35 | -- | -- |
| | Catechin | -- | -- | -- | -- | 0.40 | 0.82 |
| (C) pH-responsive thickener | Carbomer (Carbopol 981) | 0.40 | 0.40 | 0.40 | 0.40 | 0.40 | 0.40 |
| (D) Other thickeners | Sodium stearoxy PG-hydroxyethylcellulose sulfonate | 0.4 | 0.4 | 0.4 | 0.4 | 0.4 | 0.4 |
| | Dipropylene glycol[20] | 10 | 10 | 10 | 10 | 10 | 10 |
| | Preservative[21] | 0.2 | 0.2 | 0.2 | 0.2 | 0.2 | 0.2 |
| | Sodium benzoate[22] | 0.1 | 0.1 | 0.1 | 0.1 | 0.1 | 0.1 |
| | 48% sodium hydroxide | q.s. | q.s. | q.s. | q.s. | q.s. | q.s. |
| | Water | Balance | Balance | Balance | Balance | Balance | Balance |
| pH (after adjustment) | | 4.1 | 4.1 | 4.1 | 4.1 | 4.1 | 4.1 |
| Content of (A) (mass%) | | 2.00 | 2.00 | 2.00 | 2.00 | 2.00 | 2.00 |
| Total content of (A) + (B) (mass%) | | 3.00 | 2.63 | 3.00 | 2.35 | 2.40 | 2.82 |
| Mass ratio of ((A)/(B)) | | 2.00 | 3.17 | 2.00 | 5.71 | 5.00 | 2.44 |
| Viscosity at pH 4.1 (mPa·s) | | 2505 | 3550 | 3379 | 3400 | 2248 | 1811 |
| Viscosity at pH 5.0 (mPa·s) | | -- | -- | -- | -- | -- | -- |
| Viscosity change from viscosity at pH 4.1 to viscosity at pH 5.0 (times) | | -- | -- | -- | -- | -- | -- |

| | Component name | Example 38 | Example 39 | Example 40 | Example 41 | Example 42 | Example 43 |
|---|---|---|---|---|---|---|---|
| Evaluation | Scalp protection ability (%) | 31.2 | 18.6 | 18.4 | 18.2 | 30.4 | 24.4 |
| | Dyeability ($\Delta$E*) | -- | -- | -- | -- | -- | -- |
| | Application property | -- | -- | -- | -- | -- | -- |
| | Dripping property (cm) | -- | -- | -- | -- | -- | -- |

*16: Ascorbic Acid manufactured by Watanabe Chemical Co., Ltd.

*17: BHT manufactured by Nikki-Universal Co., Ltd.

*18: Nicotinic acid dl-$\alpha$-Tocopherol manufactured by Joko Pharmaceutical Co., Ltd.

*19: Purified Anhydrous Sodium Sulfite manufactured by Daito Chemical Co., Ltd.

*20: DPG-RF manufactured by ADEKA CORPORATION *21: Neolone PH 100 manufactured by The Dow Chemical Company *22: Purox S manufactured by Emerald Kalama Chemical, B.V.

From the tables above, in Examples 1 to 43 of the present invention, cosmetic compositions containing all of the pH buffer, the antioxidant and the pH-responsive thickener are used, the viscosity of the cosmetic compositions at pH 4.1 is from 1 to 30,000 mPa·s, and the cosmetic composition is applied before hair treatment, so that the scalp protection ability can be significantly improved as compared to Comparative Examples 1 to 3 in which at least one of the pH buffer, the antioxidant and the pH-responsive thickener is not added to a cosmetic composition to be used, or Comparative Example 4 in which the viscosity of the cosmetic composition at pH 4.1 is more than 30,000 mPa·s to be used. For the compositions of all Examples of the present invention, not only the scalp protection ability can be significantly improved, but also it is possible to maintain an excellent dyeing effect and maintain a good balance between an application property and a dripping property.

Comparison of Comparative Examples 1 to 3 with Examples of the present invention shows that the scalp protection ability decreases when any one of the pH buffer, the antioxidant and the pH-responsive thickener is not added to a composition to be used. As can be seen from Comparative Example 4, when the viscosity at pH 4.1 of a composition to be used is more than 30,000 mPa·s, the application property is deteriorated, and thus inconvenience is given to a user and also the scalp protection ability is reduced because the composition cannot be uniformly applied to the entire scalp.

Industrial Applicability

The hair treatment method of the present invention can be suitably used in the fields of cosmetics and the like. The hair treatment method is suitable for not only humans but also animals such as dogs and cats.

**Claims**

1. A hair treatment method comprising the following step 1 and step 2, the step 2 being carried out after the step 1:

   (step 1) applying to the scalp a cosmetic composition comprising the following components (A), (B) and (C) and having a viscosity of from 1 to 30,000 mPa·s at pH 4.1:

   (A) a pH buffer;
   (B) an antioxidant; and
   (C) a pH-responsive thickener; and

   (step 2) applying to the hair at least one hair treatment agent composition selected from the group consisting of a hair color composition, a hair bleach composition, a perm composition and a relaxer composition.

2. The hair treatment method according to claim 1, wherein a content of the component (A) in the cosmetic composition is more than 0.15 mass% based on the total amount of the cosmetic composition.

3. The hair treatment method according to claim 1 or 2, wherein a content of a surfactant in the cosmetic composition is less than 5.0 mass%.

4. The hair treatment method according to any one of claims 1 to 3, wherein a total content of the component (A) and the component (B) in the cosmetic composition is 0.2 mass% or more and 6.0 mass% or less based on the total amount of the cosmetic composition.

5. The hair treatment method according to any one of claims 1 to 4, wherein a mass ratio of the component (A) to the component (B), ((A)/(B)), is 1.0 or more and 8.0 or less.

6. The hair treatment method according to any one of claims 1 to 5, wherein the cosmetic composition further comprises a (D) thickener different from the component (C) .

7. The hair treatment method according to claim 6, wherein the component (D) is sodium stearoxy PG-hydroxyethylcellulose sulfonate.

8. The hair treatment method according to any one of claims 1 to 7, wherein a viscosity of the cosmetic composition at pH 5.0 is from 1.1 to 10 times as large as a viscosity of the cosmetic composition at pH 4.1.

9. The hair treatment method according to any one of claims 1 to 8, wherein the pH buffer has a buffering ability at least over a pH range of from 4.5 to 5.5.

10. The hair treatment method according to any one of claims 1 to 9, wherein the pH buffer exhibits a buffering ability at least over a pH range of from 4.5 to 5.5 by using an acid with a pKa (25°C) of from 4.0 to 6.5, and a salt thereof.

11. The hair treatment method according to any one of claims 1 to 10, wherein the pH buffer exhibits a buffering ability at least over a pH range of from 4.5 to 5.5 by using at least one acid selected from the group consisting of barbituric acid, succinic acid, citric acid, acetic acid, malic acid, phthalic acid and carbonic acid, and a salt thereof.

12. A cosmetic composition for protecting the scalp, comprising the following components (A), (B) and (C) and having a viscosity of from 1 to 30,000 mPa·s at pH 4.1:

    (A) a pH buffer;
    (B) an antioxidant; and
    (C) a pH-responsive thickener.

13. The cosmetic composition according to claim 12, wherein the pH buffer has a buffering ability at least over a pH range of from 4.5 to 5.5.

14. A kit comprising:

    (I) a cosmetic composition comprising the following components (A), (B) and (C) and having a viscosity of 1 to 30,000 mPa·s at pH 4.1:

        (A) a pH buffer;
        (B) an antioxidant; and
        (C) a pH-responsive thickener; and

    (II) at least one or more hair treatment agent compositions selected from the group consisting of a hair color composition, a hair bleach composition, a perm composition and a relaxer composition.

15. The kit according to claim 14, wherein the pH buffer has a buffering ability at least over a pH range of from 4.5 to 5.5.

## INTERNATIONAL SEARCH REPORT

| International application No. |
|---|
| **PCT/JP2021/027670** |

### A. CLASSIFICATION OF SUBJECT MATTER

*A61K 8/365*(2006.01)i; *A61K 8/67*(2006.01)i; *A61K 8/73*(2006.01)i; *A61K 8/81*(2006.01)i; *A61Q 5/08*(2006.01)i
FI:  A61K8/365; A61K8/67; A61K8/81; A61K8/73; A61Q5/08

According to International Patent Classification (IPC) or to both national classification and IPC

### B. FIELDS SEARCHED

Minimum documentation searched (classification system followed by classification symbols)

A61K8/365; A61K8/67; A61K8/73; A61K8/81; A61Q5/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2017-155028 A (OKADA GIKEN KK) 07 September 2017 (2017-09-07) claims, paragraphs [0005], [0015], [0017], [0021]-[0033] | 1-6, 9-11 |
| Y | | 7-8, 12-15 |
| Y | JP 2016-108296 A (KAO CORP.) 20 June 2016 (2016-06-20) paragraphs [0031], [0032] | 7, 8, 12-15 |
| Y | JP 2016-193872 A (KAO CORP.) 17 November 2016 (2016-11-17) paragraph [0104] | 7, 8, 12-15 |
| A | JP 2001-342119 A (CERAMIDE SHA KK, ORION SHOHIN KOGYO KK) 11 December 2001 (2001-12-11) entire text | 1-15 |

☐ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **14 September 2021** | **28 September 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

| Patent document cited in search report | | | Publication date (day/month/year) | Patent family member(s) | | | Publication date (day/month/year) |
|---|---|---|---|---|---|---|---|
| JP | 2017-155028 | A | 07 September 2017 | (Family: none) | | | |
| JP | 2016-108296 | A | 20 June 2016 | WO | 2016/093189 | A1 | |
| JP | 2016-193872 | A | 17 November 2016 | WO | 2016/093189 | A1 | |
| JP | 2001-342119 | A | 11 December 2001 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107837212 A

- JP 2019123710 A